# FASCICULE DE BREVET EUROPEEN

(11) **EP 2 142 248 B1**
(45) Date de publication et mention de la délivrance du brevet: **13.10.2010**
(21) Numéro de dépôt: 08788128.0
(22) Date de dépôt: 04.04.2008
(51) Int. Cl.: A61N 1/08, A61N 1/36, A61F 2/68, G06F 1/32, G06F 13/24, G06F 15/163, A61F 2/70, A61F 9/08

(54) **SYSTEME DE PROTHESE BUCCALE COMPRENANT UN DISPOSITIF D'ELECTROSTIMULATION ASSOCIE A UN DISPOSITIF D'EMISSION-RECEPTION SANS FIL**
ORALPROTHESENSYSTEM MIT EINER ELEKTROSTIMULATIONSVORRICHTUNG, DIE MT EINER DRAHTLOSEN SENDE- UND EMPFANGSVORRICHTUNG GEKOPPELT IST
ORAL PROSTHESIS SYSTEM INCLUDING AN ELECTROSTIMULATION DEVICE ASSOCIATED WITH A WIRELESS TRANSMISSION-RECEPTION DEVICE

(30) Priorité: 05.04.2007 FR 0754295
(43) Date de publication de la demande: 13.01.2010
(73) Titulaire: Université Joseph Fourier, 38041 Grenoble Cedex 9 (FR)
(72) Inventeur: COLIN, Bruno, F-34400 Saint Series (FR); PAYAN, Yohan, F-38580 Allevard (FR)
(74) Mandataire: de Beaumont, Michel
(86) Numéro de dépôt international: PCT/FR2008/050605
(87) Numéro de publication internationale: WO 2008/139099

(56) Documents cités:
- FR-A- 2 505 661
- FR-A- 2 533 719
- FR-A- 2 884 151
- US-A- 5 792 067
- US-B1- 6 598 006
- J. PATTERSON; J. DIXON: "Optimizing Power Consumption in DSP Designs" TEXAS INSTRUMENTS WHITE PAPER, [Online] septembre 2006 (2006-09), pages 1-9, XP002462011 USA Extrait de l'Internet: URL:http://focus.ti.com/lit/ml/spry089/spr y089.pdf> [extrait le 2007-12-10]

## Description

### Domaine de l'invention

La présente invention concerne la réalisation de dispositifs d'électrostimulation linguale.

### Exposé de l'art antérieur

Les dispositifs d'électrostimulation comportent une matrice d'électrodes placée dans la voûte palatine d'un individu au-dessus de la langue sous la forme d'une prothèse buccale amovible susceptible d'être fixée à la dentition du porteur. Un tel dispositif est par exemple décrit dans le brevet américain N° 6 430 450.

On a proposé d'utiliser des dispositifs d'électrostimulation linguale dans de nombreuses applications. Ces applications visent notamment à accroître l'autonomie d'une personne handicapée de façon permanente ou temporaire en compensant une déficience temporaire ou permanente. Notamment de tels dispositifs sont aptes à fournir au porteur des informations que son corps ne lui fournit pas. Ces applications comprennent une rééducation consécutive à un traumatisme accidentel, maladif ou chirurgical d'une articulation ou d'un segment corporel.

Ces applications sont rendues possibles car la langue du porteur vient à intervalles réguliers - par mouvement réflexe - au contact de la matrice fixée au palais, ce qui permet de fournir des informations au porteur. Par exemple, comme le décrit le brevet américain N° 6 430 450, une déficience visuelle est compensée en fournissant des informations visuelles d'une caméra embarquée vers le dispositif d'électrostimulation linguale.

De façon symétrique, on a proposé d'utiliser le dispositif d'électrostimulation linguale pour que le porteur puisse interagir avec son environnement. Ainsi, le porteur pourrait commander le déclenchement d'une alarme ou les déplacements d'un équipement motorisé tel qu'un lit ou un fauteuil roulant, ou commander un équipement domotique tel qu'un éclairage, un téléphone, une porte ou un ordinateur.

On a également proposé qu'un même dispositif d'électrostimulation linguale soit utilisé tant pour fournir des informations de l'extérieur au porteur que pour lui permettre d'interagir avec son environnement. Ainsi, le brevet américain N° 6 430 450 propose que le porteur puisse à partir des données visuelles transférées sur sa langue provoquer le déclenchement d'un actionneur tel qu'une main robotisée.

Par ailleurs, on a proposé que les opérations de transfert d'informations soient effectuées de façon non filaire par un dispositif d'émission/réception sans fil embarqué dans la prothèse comportant le dispositif d'électrostimulation linguale. Ainsi, le brevet américain N° 6 430 450 prévoit que les informations en provenance de la caméra soient fournies au dispositif d'électrostimulation par émission/réception sans fil.

Le brevet US 5 792 067 montre un système selon le préambule de la revendication 1 et comprend un seul microprocesseur.

Toutes ces propositions ont été émises alors qu'il n'existait pas de dispositif d'électrostimulation linguale comportant une fonction d'émission/réception sans fil et étaient basées sur l'hypothèse que la réalisation de tels dispositifs serait aisée.

En pratique, la réalisation de tels dispositifs se heurte à de nombreuses difficultés et contraintes. Parmi ces contraintes, on notera la nécessité d'une autonomie relativement élevée de l'ordre d'au moins une journée d'utilisation. Une autre contrainte réside dans l'encombrement. Celui-ci doit être réduit pour que le dispositif d'émission/réception puisse être contenu dans la même prothèse buccale que le dispositif d'électrostimulation linguale. Une autre contrainte qui se combine à la contrainte d'encombrement réduit est que le dispositif doit présenter une dissipation thermique extrêmement réduite. Il faut en effet éviter que la voûte palatine, la langue, les gencives et/ou les dents du porteur ne soit exposées de façon prolongée à de la chaleur.

Un mode de réalisation de la présente invention vise à proposer un système de prothèse buccale comportant un dispositif d'électrostimulation linguale associé à un émetteur récepteur sans fil.

Un mode de réalisation de la présente invention vise à proposer un tel système susceptible de fonctionner avec une faible consommation d'énergie.

Un mode de réalisation de la présente invention vise à proposer un tel système qui présente un encombrement restreint.

Un mode de réalisation de la présente invention vise à proposer un tel système qui fonctionne en radiofréquence.

### Résumé de l'invention

Pour atteindre tout ou partie de ces objets ainsi que d'autres, un mode de réalisation prévoit un système de prothèse buccale comprenant un dispositif d'électrostimulation linguale associé à un dispositif d'émission-réception sans fil et comportant deux microcontrôleurs, un premier microcontrôleur dédié aux opérations d'émission et de réception, et un second microcontrôleur dédié aux opérations de commande d'une matrice d'électrostimulation.

Selon un mode de réalisation, les microcontrôleurs sont reliés par une interface série du type émetteur-récepteur universel asynchrone.

Selon un mode de réalisation, les microcontrôleurs sont des microcontrôleurs propres à passer d'un mode de veille à un mode actif en un temps inférieur à 5 µs.

Selon un mode de réalisation, les microcontrôleurs sont des microcontrôleurs à très faible courant moyen pendant une phase d'attente comportant des moments très courts d'activation périodique du dispositif de réception.

Selon un mode de réalisation, le courant moyen des microcontrôleurs est inférieur à 15 µA pendant la phase d'attente, les moments d'activation du dispositif de réception durant au plus 1 milliseconde et apparaissant une fois par seconde.

La présente invention est applicable à un procédé d'électrostimulation utilisant un système selon l'un quelconque des modes de réalisation précédents, chacun des microcontrôleurs étant en phase d'attente pendant laquelle il initie des contrôles périodiques d'apparition d'une interruption jusqu'à l'apparition d'une interruption.

### Brève description des dessins

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 est un diagramme illustrant schématiquement divers éléments d'un système de prothèse buccale selon un mode de réalisation de la présente invention ;
la figure 2 est un chronogramme illustrant une phase d'un mode de fonctionnement d'un système selon un mode de réalisation de la présente invention ; et
les figures 3A et 3B sont des chronogrammes illustrant une autre phase d'un mode de fonctionnement d'un système selon un mode de réalisation de la présente invention.

### Description détaillée

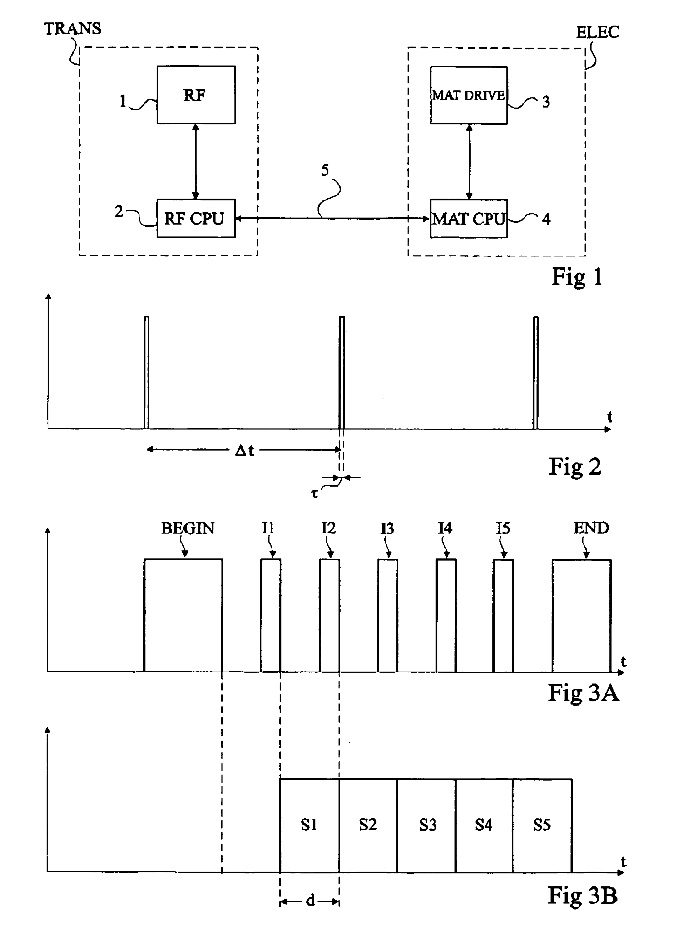

La figure 1 illustre un diagramme schématique d'un système de prothèse buccale comprenant un dispositif d'électrostimulation linguale associé à un dispositif d'émission et de réception sans fil selon un mode de réalisation de la présente invention.

Le système selon un mode de réalisation de la présente invention comporte un bloc d'émission/réception TRANS comprenant un émetteur-récepteur radiofréquence RF 1 associé à un processeur RFCPU 2 dédié aux opérations d'émission/réception. Le système comporte également un bloc d'électrostimulation comprenant une matrice d'électrodes associée à des.éléments de commande de la matrice d'électrostimulation MATDRIVE 3 pilotée par un processeur dédié MATCPU 4. Les deux processeurs 2 et 4 sont associés par l'intermédiaire d'une interface série du type émetteur-récepteur universel asynchrone (UART) 5.

De façon surprenante, recourir à deux processeurs placés dans la prothèse buccale permet d'obtenir une plus faible consommation que l'utilisation d'un processeur plus puissant propre à gérer tant les opérations d'émission-réception que les opérations de commande du dispositif d'électrostimulation. Comme cela ressortira de la description ci-après d'un mode de fonctionnement du système selon un mode de réalisation de la présente invention, cela est notamment dû au fait que chaque processeur peut avantageusement être placé dans des modes de faible consommation indépendamment l'un de l'autre.

Un mode de fonctionnement d'un système selon un mode de réalisation de la présente invention est décrit ci-après en relation avec les chronogrammes schématiques des figures 2, 3A et 3B. La figure 2 illustre une phase de veille du système. Les figures 3A et 3B illustrent une phase active du système lorsque le système reçoit des données de l'extérieur.

Tant qu'aucune requête d'activation de la matrice d'électrostimulation n'est envoyée par un dispositif externe, le système est en mode d'attente. Pendant un tel mode, le bloc d'émission-réception TRANS devient actif périodiquement, comme l'illustre la figure 2A, par exemple toutes les secondes. Pendant chacune de ces phases d'activité provoquée par le processeur 2, le bloc TRANS contrôle si aucune requête d'activation n'est émise par un dispositif externe. En l'absence de telle requête, la durée d'activité du bloc TRANS est inférieure à une milliseconde. Tant qu'aucune requête n'est détectée, la consommation moyenne est très faible, par exemple inférieure à 15 microampères avec des processeurs classiques.

Les figures 3A et 3B illustrent l'activité du bloc d'émission TRANS et du bloc d'électrostimulation ELEC pendant une phase active du système. Comme l'illustre la figure 3A, au cours d'un contrôle périodique, séparé d'une seconde du contrôle précédent, on suppose que le bloc TRANS détecte une trame d'activation BEGIN. Le processeur 2 provoque alors un passage en mode actif du bloc TRANS ainsi que du bloc ELEC par l'intermédiaire de l'interface 5. Le bloc TRANS reçoit alors une succession d'instructions d'électrostimulation comportant l'emplacement de l'électrode à activer ainsi que le cas échéant des instructions de durée et d'intensité de stimulation. Par exemple, la figure 3A illustre cinq instructions successives I1, I2, I3, I4 et I5. A chaque instruction I correspond une électrostimulation S d'une électrode de la matrice. De préférence, comme l'illustre la comparaison des figures 3A et 3B, l'intervalle de temps séparant la réception de deux instructions successives est optimisé de façon que les électrostimulations correspondantes se suivent immédiatement. Ainsi, la fin de la réception de la première instruction I1 est séparée de la fin de la réception de la deuxième instruction I2 d'un intervalle de temps d, par exemple de l'ordre de 100 millisecondes, nécessaire au traitement de l'instruction I1 par le processeur de réception 2, son transfert au processeur de stimulation 4, son traitement par ce dernier et son transfert au porteur sous forme d'une électrostimulation S1.

Lorsque la dernière instruction I5 a été émise par un dispositif externe, celui-ci interrompt toute transmission, et le processeur de réception 2 détecte l'absence de nouvelles instructions et place de nouveau le système en état de veille.

Selon une variante, après émission de la dernière instruction I5, le dispositif externe émet une trame de fin END qui indique au processeur que la transmission est achevée.

De préférence, lorsque la transmission est achevée, avant de retourner à l'état de veille, le bloc TRANS émet un message d'accusé-réception.

On a décrit précédemment le fonctionnement du système en réception. Toutefois, l'homme de l'art comprendra que le fonctionnement en transmission est symétrique. Ainsi, le processeur d'électrostimulation 4 contrôle à intervalles réguliers pendant une phase de veille si des instructions proviennent du porteur par l'intermédiaire du dispositif 3. Si, au cours d'un de ces contrôles, il détecte que le porteur souhaite émettre des instructions, il provoque l'activation du bloc TRANS par l'interface 5.

Selon un mode de réalisation, les processeurs 2 et 4 sont des microcontrôleurs à très faible consommation tout en ayant des temps d'activation et de désactivation très courts. Ainsi, les processeurs doivent pouvoir passer d'un état de veille à un état actif en moins de 5 µs tout en ayant un courant moyen inférieur à 15 µA, de préférence d'au plus 12 pA, pendant les phases très courtes d'activation périodique du bloc émetteur-récepteur pendant la phase d'attente. Comme cela a été exposé, de telles phases apparaissent par exemple toute les secondes et sont aussi courtes qu'une milli-seconde. Par exemple, ce sont des microcontrôleurs du type MSP 430 commercialisés par la société Texas Instruments ou un microcontrôleur équivalent du même fabricant ou d'un autre fabricant. De façon générale, le système selon un mode de réalisation de la présente invention peut utiliser tout type de microcontrôleur présentant une tension de fonctionnement basse, une faible consommation dans un mode de fonctionnement comportant la gestion de réveil périodique, une faible consommation en mode actif et la possibilité de passer d'un mode inactif à un mode actif dans un temps aussi réduit que possible, de préférence inférieur à 5 microsecondes. Le processeur 4 de gestion de la matrice d'électrostimulation linguale doit également être un processeur de faible encombrement qui présente une consommation très faible en mode de veille. De préférence, afin de réduire la consommation de fonctionnement, le système est régulé à l'aide d'un régulateur de 2,3 V à très faible consommation du type LDO de l'anglais low drop out voltage. Un tel régulateur permet d'assurer une plage de fonctionnement suffisamment importante sans variation des performances du système d'émission-réception à radio fréquence. En effet, les performances sont stables et garanties tant que la tension de la pile est comprise entre sa tension nominale, de l'ordre de 2,5 volts, et la tension du régulateur.

Le dispositif de commande de la matrice d'électrostimulation 3 comporte notamment des convertisseurs numérique/analogique et des multiplexeurs. De préférence, le choix des composants est effectué de façon que ce système peut être rendu inactif très rapidement et passe dans un mode très basse consommation, de préférence inférieur à un microampère.

Bien entendu, la présente invention est susceptible de diverses variantes et modifications qui apparaîtront à l'homme de l'art. En particulier, le protocole de transmission n'a été décrit que de façon très simplifiée. L'homme de l'art saura choisir et adapter un protocole particulier en fonction de l'application visée. De même, l'homme de l'art saura choisir les différents éléments de la prothèse buccale en fonction des enseignements de la présente demande et de l'application visée.

## Revendications

1. Système de prothèse buccale comprenant un dispositif d'électrostimulation linguale associé à un dispositif d'émission-réception sans fil, **caractérisé en ce que** le système comprend deux microcontrôleurs, un premier microcontrôleur (2) dédié aux opérations d'émission et de réception, et un second microcontrôleur (4) dédié aux opérations de commande d'une matrice d'électrostimulation.

2. Système selon la revendication 1, dans lequel les microcontrôleurs (2, 4) sont reliés par une interface série du type émetteur-récepteur universel asynchrone (5).

3. Système selon la revendication 1 ou 2, dans lequel les microcontrôleurs (2, 4) sont des microcontrôleurs propres à passer d'un mode de veille à un mode actif en un temps inférieur à 5 µs.

4. Système selon la revendication 3, dans lequel les microcontrôleurs (2, 4) sont des microcontrôleurs à très faible courant moyen pendant une phase d'attente comportant des moments très courts d'activation périodique du dispositif de réception.

5. Système selon la revendication 4, dans lequel le courant moyen des microcontrôleurs (2, 4) est inférieur à 15 µA pendant la phase d'attente, les moments d'activation du dispositif de réception durant au plus 1 milliseconde et apparaissant une fois par seconde.

## Claims

1. An oral prosthesis system comprising an electrical tongue stimulation unit associated with a wireless transceiver device, **characterized in that** the system comprises two microcontrollers, a first microcontroller (2) dedicated to transmit and receive operations, and a second microcontroller (4) dedicated to the control of an electrical stimulation array.

2. The system of claim 1, wherein the microcontrollers (2, 4) are connected by a serial interface of universal asynchronous transceiver type (5).

3. The system of claim 1 or 2, wherein the microcontrollers (2, 4) are microcontrollers capable of switching from a standby mode to an active mode within a time shorter than 5 µs.

4. The system of claim 3, wherein the microcontrollers (2, 4) are microcontrollers of very low average current during a standby phase comprising very short periods of periodic activation of the receive device.

5. The system of claim 4, wherein the average current of the microcontrollers (2, 4) is lower than 15 µA during the standby phase, the periods of activation of the receive device lasting for at most 1 millisecond and occurring once per second.

## Patentansprüche

1. Oralprothesensystem, welches eine elektrische Zungenstimulationseinheit aufweist, die mit einer drahtlosen Sende- und Empfangs- bzw. Transceivervorrichtung assoziiert ist, **dadurch gekennzeichnet, dass** das System zwei Mikrocontroller aufweist, wobei ein erster Mikrocontroller (2) für die Sende- und Empfangsfunktionen bestimmt ist, und wobei ein zweiter Mikrocontroller (4) für die Steuerung einer Elektrostimulationsanordnung bestimmt ist.

2. System nach Anspruch 1, wobei die Mikrocontroller (2, 4) über eine serielle Schnittstelle vom Typ einer universellen asynchronen Sende- und Empfangsvorrichtung bzw. eines universellen asynchronen Transceivers (5) verbunden sind.

3. System nach Anspruch 1 oder 2, wobei die Mikrocontroller (2, 4) Mikrocontroller sind, welche in der Lage sind, innerhalb eines Zeitraums von weniger als 5 µs von einem Standby-Modus zu einem aktiven Modus zu schalten.

4. System nach Anspruch 3, wobei die Mikrocontroller (2, 4) Mikrocontroller mit sehr niedrigem mittleren Strom während einer Standby-Phase sind, welche sehr kurze Perioden einer periodischen Aktivierung der Empfangsvorrichtung aufweist.

5. System nach Anspruch 4, wobei der mittlere Strom der Mikrocontroller (2, 4) während der Standby-Phase niedriger als 15 µA ist, wobei die Perioden der Aktivierung der Empfangsvorrichtung maximal 1 Millisekunde dauern und einmal pro Sekunde auftreten.
